# EUROPEAN PATENT APPLICATION

(11) **EP 0 796 837 A1**
(43) Date of publication of application: **24.09.1997**
(21) Application number: 97104879.8
(22) Date of filing: 21.03.1997
(51) Int. Cl.: C07C 51/265, C07C 63/26

(54) **Process for producingan aromatic carboxylic acid**

(30) Priority: 22.03.1996 US 620201
(71) Applicant: PRAXAIR TECHNOLOGY, INC., Danbury, CT 06810-5113 (US)
(72) Inventor: Roby, Anne Katherine, Golden's Bridge, New York 10526 (US); Kingsley, Jeffrey Paul, East Amherst, New York 14051 (US)
(74) Representative: Schwan, Gerhard, Dipl.-Ing.

(57) **Abstract**

The invention involves an oxidation process for making an aromatic carboxylic acid, wherein the amount of water required has been optimized. As a result, the decreased molar flow rate of vapor and decreased boiling temperature improve the production efficiency of a high quality product.

## Description

### FIELD OF THE INVENTION

This invention relates to a method for producing terephthalic acid (TA) and more particularly to a method for producing TA wherein the amount of water used in the reaction is optimized so as to improve production efficiency.

### BACKGROUND

Terephthalic acid (TA) is produced commercially by the catalytic oxidation of p-xylene in acetic acid. This process is known as the Amoco or Mid-Century process. A typical air-based process for producing TA is described in U.S. Patent 3,089,906 (1963) and more recently in U.S. Patent 5,081,290 (1992). The conversion of p-xylene is very high (>99%) with selectivity to TA of approximately 98%. The reaction is conducted in continuously stirred tank reactors. The ratio of solvent to reactant is typically two to six volumes of solvent per volume of reactant (2:1 to 6:1). The reaction product is a solid which precipitates out of solution, forming a slurry mixture with the solvent.

The oxidation reaction is exothermic with a heat of reaction of 326 kcal/gmol. The heat of reaction is removed by the evaporation of solvent and by-product water. The pressure of the reactor is fixed such that a liquid phase is maintained in the reaction zone, approximately between 100 and 300 psig. The temperature of the process is set by the boiling range of the mixture, typically between 170°C and 225°C. The vapor is condensed and returned to the reactor. In the commercial process, the water is removed from the condensate before returning to the reactor.

Compressed air is sparged into the bottom of the reactor. Oxygen from the air dissolves into the liquid phase and reacts with the p-xylene to produce TA. Intermediate oxidation products and by-products are also formed in quantities which depend on reaction conditions. The residence time is typically one hour.

The transfer of oxygen from the bubble to the liquid is critical to the formation of high quality product. At some conditions, the overall reaction rate is controlled by the rate of oxygen mass transfer, which depends on the concentration of oxygen in the bubble and the total pressure of the process. More particularly, as solvent evaporates into the bubble, the oxygen concentration is diminished and the mass transfer rate decreases. At all conditions, any region of low oxygen concentration could cause the formation of undesirable coupled by-products.

As the air bubbles are dispersed and circulated throughout the liquid phase by an agitator, the oxygen concentration in the bubbles decreases as the oxygen dissolves and reacts. The air bubbles disengage from the liquid phase and collect in the top of the reactor to form a continuous gas phase. This waste gas must be vented in order to make room for fresh air feed and to maintain adequate gas hold-up to promote oxygen transfer from the gas to the liquid phase.

To avoid the possibility of fire or explosion, the oxygen concentration in the gas space at the top of reactor must be maintained below the flammable limit. For practical purposes, the oxygen concentration must be maintained at less than 8-9% by volume [U.S. Patent 3,092,658]. More typically, the oxygen concentration in the gas space is maintained below 5% by volume to provide a safe margin below the flammable limit. Thus in a well-mixed, stirred tank reactor, the average concentration of oxygen in the circulating air bubbles must be below 5% in order to insure that the average concentration of oxygen in the gas which collects in the headspace is nonflammable.

The oxygen concentration in the gas space is a function of the rate at which air is fed into the reactor and the rate of consumption of oxygen from the air by reaction. The rate of reaction and, therefore, the TA production rate per unit of reactor volume, increases with temperature, pressure, oxygen concentration in the gas phase, p-xylene concentration, promoter concentration and catalyst concentration. Since the concentration of dissolved oxygen in the liquid phase and, hence, the reaction rate of oxygen, is proportional to the oxygen concentration in the gas phase, for a given set of reaction conditions, the 5% oxygen restriction effectively limits the oxygen reaction rate.

By maintaining a high reaction rate, the formation of undesirable intermediates can be minimized. The rate of reaction increases with increasing temperature. Typically, the presence of intermediates is minimized by maintaining a high reaction temperature. However, the rate of combustion of the solvent has been observed to increase with increasing temperature. Therefore, the use of high temperature to increase reaction rate has a significant operating cost associated with it due to loss of solvent. In addition, there are environmental concerns as the aforementioned air-based terephthalic acid production processes produce CO₂, CO, methyl bromide and methyl acetate by-product gases.

The effect of water as a variable in air-based reactions has been investigated in the prior art. For example, Lindahl et al in U.S. Patent 4,835,307 teach that water content in TA production is one of several variables to consider when attempting to minimize by-product formation. Specifically, they teach that the amount of water in the reactor should be between 5% and 20% by weight. Tamaru et al teach that the water content in the reactor may be controlled to a level of 5 to 15 wt.%, but there is no suggestion as to why this range is important. Finally, Roffia et al, in *Oxidation Communications* **8**, Nos. 1-2, teach that water concentration in the reaction medium has an effect on kinetics, selectivity, acid quality and solvent and catalyst consumption. Specifically, they measure the effects of a water concentration in the reactor of between 6% and 18% by weight. Figure 10 of the reference shows that when water concentration is increased, the rate of oxidation decreases. In light of this, Roffia teaches that when adding water, the temperature of the reaction must be increased so as to preserve the rate of oxidation. As indicated above, increasing the temperature of the reaction results in increased costs due to solvent loss.

We have previously proposed, in U.S. Patent 5,371,283, an oxygen based process which uses pure or nearly pure oxygen (by "pure or nearly pure" we mean gas having at least about 50 vol.% oxygen, preferably greater than 90 vol.% oxygen) in place of air to enhance reaction rate and product quality. In this reaction of p-xylene to terephthalic acid, the reaction rate is kept high by increasing the partial pressure of the oxygen in the bubble. The rate of transfer of oxygen into the liquid for reaction is thus increased, increasing the overall rate of reaction. Therefore, an equivalent reaction rate can be achieved at a lower temperature than with the air-based reaction.

In the present invention, we have found that the amount of water used in the reaction has an effect on the concentration of oxygen in the bubble, and as such there is an optimal amount which may be determined. We believe that this relationship has not been heretofore explored, as in the air-based reaction the effects of water on oxygen concentration are significantly dampened due the presence of nitrogen. As such optimization of water content beyond those ranges previously disclosed is not possible.

### OBJECT OF THE INVENTION

It is therefore an object of the invention to determine an optimal amount of water to be used in an oxygen based TA production process.

### SUMMARY OF THE INVENTION

This invention comprises a process for making an aromatic carboxylic acid, said process comprising the steps of:
a) providing a body of liquid contained within a reactor vessel, said body of liquid comprising an organic solvent, water in an amount of about 6 to about 10.5 wt.%, at least one heavy metal catalyst, and a bromine initiator;
b) adding an aromatic alkyl reactant to said body of liquid;
d) adding pure or nearly pure oxygen to said body of liquid;
e) removing the heat of the reaction due to the oxidation of said aromatic alkyl by evaporative cooling upon evaporation of volatile organic materials and water present in said body of liquid;
f) recovering aromatic carboxylic acid product.

### BRIEF DESCRIPTION OF THE DRAWINGS

Other objects, features and advantages will occur to those skilled in the art from the following description of preferred embodiments and the accompanying drawings, in which:
Figure 1 is a graph showing the effect of water concentration on the total number of moles of solvent/water mixture evaporated.
Figure 2 is a graph which illustrates the difference in the affect of dilution on the oxygen bubble and an air bubble containing the same amount of oxygen, but also including the diluent nitrogen.
Figure 3 is a graph showing the relationship between the water concentration and the boiling point of the solvent/water mixture.
Figure 4 is a graph showing the relationship between the water concentration and the generation of COₓ.

### DETAILED DESCRIPTION OF THE INVENTION

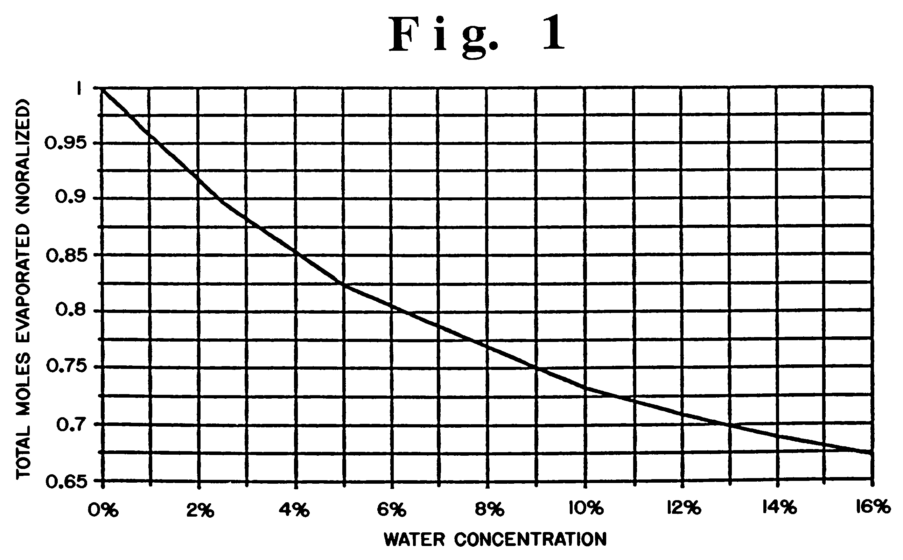

In our invention, optimizing the content of water in the reaction mixture is used to maintain a high concentration of oxygen in the bubble. Critical to this concept, is an understanding of the effect of water on oxygen concentration in the bubble. As solvent (typically acetic acid) and water vaporize into the oxygen bubble, the concentration of oxygen is diminished. Since the latent heat of vaporization of water is much greater than that of acetic acid (8.4 kcal/gmol and 5.1 kcal/gmol, respectively), the total number of moles of the mixture evaporated is much lower. This general effect is illustrated in Figure 1 which shows that as the concentration of water is increased, the relative number of moles of solvent and water required to remove the heat of reaction is reduced. This results in a decreased amount of solvent/water in the bubble, and consequently, an increase in the concentration of oxygen in the bubble. The net result is an increase in mass-transfer and reaction rate. This effect is not observed for air based reactions as other components in air (N₂ for example) dilute the oxygen concentration in the bubble. It should be noted that the effect of water on the oxidation rate of the oxygen-based reaction is opposite to that shown by Roffia et al with respect to the air-based reaction. In that case, the only affect of water which is observed it the negative affect which water has on the kinetic rate due to catalyst deactivation.

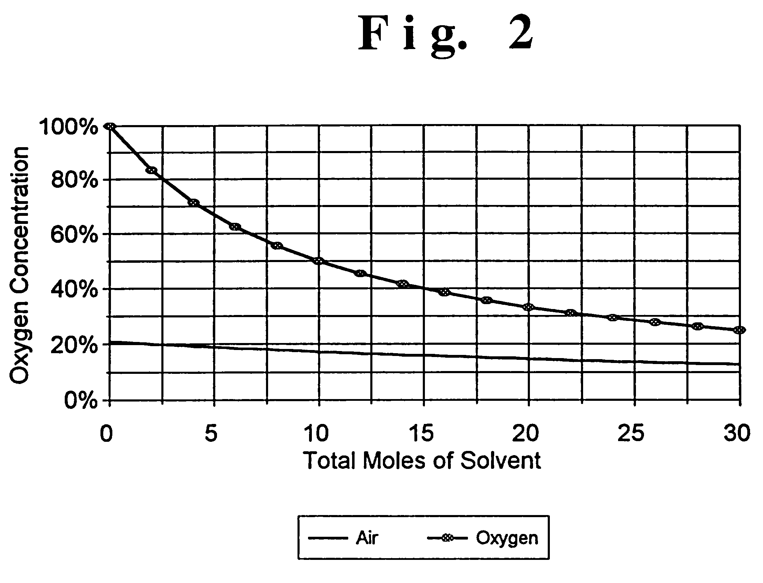

Figure 2 illustrates the difference in the affect of dilution on the oxygen bubble and an air bubble containing the same amount of oxygen, but also including the diluent nitrogen. The oxygen concentration in the oxygen bubble is decreased from 100% to 25% over the range shown. In contrast, over the same range, the concentration of oxygen in the air bubble is decreased from 21% to 13%.

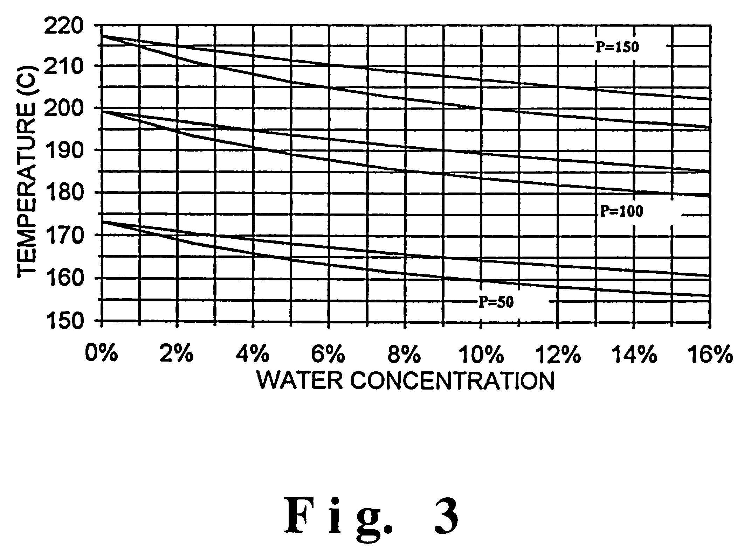

The presence of water in the reaction mixture also reduces the boiling point of the mixture. In the air-based case, this is undesirable since a higher temperature is used to increase the reaction rate. However, as mentioned above, higher temperature also increases the rate of solvent loss. Therefore, increasing the water content of the oxygen-based reaction mixture can reduce solvent loss without sacrificing reaction rate. The effect of water on the boiling point for three reaction pressures is illustrated in Figure 3, wherein it is shown that the greater the water concentration, the lower the boiling point. For each pressure, the top curve is the dew point curve, the bottom curve is the bubble point curve.

Because the reaction is run at the boiling point of the mixture, we are automatically removing heat from the reaction via evaporation, thus in a particularly preferred embodiment, the TA production reaction takes place in a manner which enables evaporative cooling to be employed, particularly through the advantageous use of a modified LOR process and system, hereinafter referred to as an evaporatively cooled LOR. The details of this process and system are disclosed in co-pending U.S. application, 08/241,438, herein incorporated by reference.

In the evaporatively cooled LOR process, oxygen is added to the reactor vessel at a point of high turbulence within or just below the hollow draft tube rather than elsewhere in the body of the solvent/reactant mixture. Injection of oxygen at such a point is important to the desired consumption of oxygen. The initially high concentration of oxygen in the gas phase at the point of injection serves to enhance the mass transfer rate of the oxygen into this region of the liquid reactant, which would otherwise be oxygen depleted due to the rapid rate of the oxidation reaction.

The practice of the invention enables a rapid rate of oxygen consumption to be achieved such that a very high oxygen use efficiency, i.e., at least 75% and preferably 90% or more, is obtained upon the first injection of pure or nearly pure oxygen into the reactor. In the practice of this embodiment the reaction of p-xylene with oxygen is greater than about 95%. That is, less than about 5% of the oxygen that is fed to the reactor is vented unreacted.

With respect to evaporative cooling, the following is noted. In the TA production operation, a significant amount of volatile organic material and water evaporate from the reaction mixture. The vent gases are cooled and the condensibles therefrom are returned to the reactor. A portion of the vent flow is desirably diverted for gas analysis of carbon dioxides and oxygen.

The advantages of the evaporative LOR will herein be described. While the TA product is obtained in the solid phase, the use of the evaporative LOR avoids the practical operating problems associated with the common use of direct cooling heat exchange surfaces for removing the heat of the oxidation reaction that result from TA and other solids precipitation on the heat transfer surfaces of cooling coils and the like. Thus, the safe and efficient use of pure or nearly pure oxygen for the p-xylene oxidation reaction can conveniently be carried out using evaporative cooling to remove the heat of reaction generated during the oxidation reaction.
In the LOR process and system, as employed in the practice of the invention, pure or nearly pure oxygen is used instead of air. This obviates the potential for fire or explosion and allows for desirable operating conditions which serve to minimize the amount of undesired byproducts present in the terephthalic acid product. In addition, the amount of vent gas to be treated is minimized. The practice of the invention enables a rapid rate of oxygen consumption to be achieved such that a very high oxygen use efficiency, i.e., at least 75% and preferably 90% or more, is obtained upon the first injection of pure or nearly pure oxygen into the reactor. In the practice of this embodiment the reaction of p-xylene with oxygen is greater than about 95%. That is, less than about 5% of the oxygen that is fed to the reactor is vented unreacted. In less preferred embodiments, the process of the invention can be carried out in conventional reactor vessels, and we believe that the optimal water concentration in the alternative reactor configuration would be identical to that required in the LOR case. Further, the substitution of oxygen for air in the oxidation of hydrocarbons could be made with conventional reactor designs, and these reactors could be run at the boiling point of the reaction mixture, thus removing the heat of reaction from the mixture with evaporative cooling. Under such conditions, many of the advantages observed with oxygen based processing, i.e., increased reaction rate, decreased vent flow, reduction in byproduct formation, are realized.

However, because the flow patterns are different in conventional systems, more of the undissolved oxygen escapes into the overhead gas phase. Therefore, the conventional system is less oxygen efficient than the LOR embodiment. In order to be economically feasible, the impeller used in such embodiments must be efficient in oxygen use by distributing the oxygen as very small bubbles and promoting a longer mean residence time for the oxygen bubbles in the liquid phase

As indicated above, in a conventional reactor there would be excess oxygen in the overhead gas phase. As a result a large amount of nitrogen or other inert vent gas must be passed to the overhead gas phase in order to avoid safety problems associated with the presence of such excess oxygen. The additional cost of such nitrogen as well as the cost of additional oxygen or other gas could well render this embodiment uneconomical from a practical operating viewpoint.

It should be noted that in addition to the physical effects which water has on the process conditions, water can also affect the chemistry of the reaction. Unfortunately, high water content can deactivate the heavy metal catalysts. Water content of approximately 20-25% is known to have this affect in the air-based oxidation, and we have found similar effects in the oxygen based reaction at about 18% by weight. Based upon these effects and upon our research, we have found that the optimum water content in the reactor for an oxygen-based reaction is about 6 to about 10.5% by weight, preferably about 8 to about 10 wt.%.

This range is considerably more narrow than that proposed for air-based reactions. We believe that part of the reason for this is that in the air-based process, the physical affects of water are substantially damped out and are not detectable due to the influence of the large nitrogen gas flow resulting from the presence of nitrogen in air. As such, optimization of the amount of water, beyond that which has been heretofore disclosed is not possible.

In the practice of the invention, a preferred solvent:reactant ratio is from about 1:1 to about 8:1 on a volume:volume basis. The preferred catalyst loadings should be within the following ranges: cobalt (400-700ppm), manganese (800-1700ppm), and bromine (500-1200ppm). The total loading should be between 500-3000ppm, and the Co:Mn ratio should be from 1:10 to 10:1. A preferred residence time for the liquid is about 60 minutes, though a time between 30 and 90 minutes is suitable. The operating temperature is generally between about 170°C and about 190°C, preferably 180°C to 190°C. The operating pressure is about 90-300 psig, preferably 100-125 psig, most preferably 115 psig. The preferred hydrocarbon reactant feed concentration varies between about 8.9% and about 14.2%. The oxidant should be pure or nearly pure oxygen.

The Examples presented below are presented for illustrative or comparative purposes and are not intended to be limiting.

### Examples

Terephthalic acid was produced by the oxidation of p-xylene in the evaporatively cooled LOR. The oxidation was conducted at a pressure of 115 psig and at temperatures between 170 and 190°C. The catalyst loadings varied within the following ranges: cobalt (450-700ppm), manganese (800-1700ppm), and bromine (500-1200ppm). The hydrocarbon reactant feed concentration varied between 9% and 14%. The oxidant was pure oxygen.

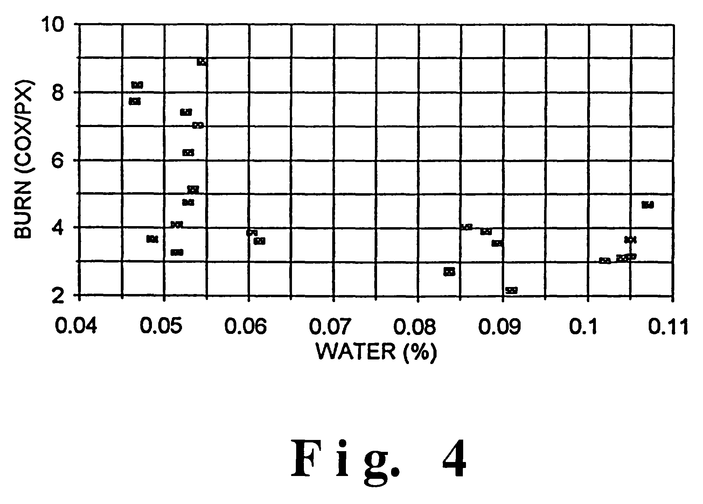

The effect of the water content on the generation of carbon oxides is illustrated in Figure 4. As can be seen, the amount of water has a significant effect upon COₓ generation. We project that the optimal water concentration in the reactor for these conditions is about 6 to about 10.5 wt.%, preferably about 8 to about 10 wt.%. At higher water concentrations, the affect of catalyst deactivation becomes evident. The positive affect of increased oxygen concentration in the bubble on oxygen mass transfer is overwhelmed by a slowing of the kinetic rate of reaction. The kinetic rate becomes the controlling rate and any additional water simply decreases the observed reaction rate.

The substitution of oxygen for air in the oxidation of hydrocarbons could be made with conventional reactor designs. In addition, these reactors could be run at the boiling point of the reaction mixture, thus removing the heat of reaction from the mixture with evaporative cooling. The advantages observed with the oxygen based process would be realized, i.e., reduction in byproduct formation. The optimal water concentration in the alternative reactor configuration would be identical to the LOR case. However, to avoid safety problems associated with excess oxygen in the gas space of such reactors, a large nitrogen flood to the gas space must be provided. The additional costs of nitrogen could very well make this embodiment of the invention uneconomical. In order to be economically feasible, the impeller used in such embodiments must be efficient in oxygen use by distributing the oxygen as very small bubbles and promoting a longer mean residence time for the oxygen bubbles in the liquid phase.

Since the invention involves the use of a co-solvent (water) which has a lower boiling point than acetic acid and a comparatively high latent heat, a substitute solvent for acetic acid could be used to achieve the same effect. With the existing catalyst system, pure water is not a suitable solvent. However, an acidic organic solvent having a latent heat which is higher than water and a boiling point which is lower than water and in which both p-xylene and water are sufficiently soluble and terephthalic acid is relatively insoluble may be used in the invention.

Those skilled in the art will appreciate that various changes and modifications can be made in the details of the invention without departing from the scope thereof as recited in the appended claims. For example, the above process is suitable for the oxidation of any other organic chemical whose oxidation produces a solid as a product or by-product. Such chemicals include, but are not limited to toluene, meta- and ortho-xylene and trimethylbenzenes. The resultant products include, but are not limited to benzoic acid, orthophthalic acid, isophthalic acid and benzenetricarboxylic acids. In addition to the production of TA, the production of isophthalic acid and 2,6-dicarboxynaphthalene are logical extrapolations of the technology.

Specific features of the invention are shown in one or more of the drawings for convenience only, as each feature may be combined with other features in accordance with the invention. Alternative embodiments will be recognized by those skilled in the art and are intended to be included within the scope of the claims.

## Claims

1. A process for making an aromatic carboxylic acid, said process comprising the steps of:
a) providing a body of liquid contained within a reactor vessel, said body of liquid comprising an organic solvent, water in an amount of about 6 to about 10.5 wt.%, at least one heavy metal catalyst, and a bromine initiator;
b) adding an aromatic alkyl reactant to said body of liquid;
c) adding pure or nearly pure oxygen to said body of liquid;
d) removing the heat of the reaction due to the oxidation of said aromatic alkyl by evaporative cooling upon evaporation of volatile organic materials and water present in said body of liquid;
e) recovering aromatic carboxylic acid product.

2. The process of claim 1, wherein the aromatic carboxylic acid is terephthalic acid, isophthalic acid or 2,6-dicarboxynaphthalene.

3. The process of claim 1, wherein the heavy metal catalyst is selected from the group consisting of cobalt and manganese.

4. The process of claim 1, wherein the solvent is acetic acid.

5. The process of claim 1, wherein the solvent:reactant ratio is from about 1:1 to about 8:1 on a volume:volume basis.

6. The process of claim 1, wherein the reactor vessel is an evaporatively cooled liquid oxygen reactor.

7. The process of claim 1, wherein the reaction temperature is 170°C to 190°C.

8. The process of claim 1, wherein the pressure is 90-300 psig.

9. The process of claim 1, wherein the residence time of the oxygen-aromatic alkyl mixture in the reactor vessel is about 60 minutes.

10. A solvent system comprising an acidic organic solvent in which both p-xylene and water are soluble and terephthalic acid is insoluble and having a lower boiling point and a higher latent heat than acetic acid.
